# EUROPÄISCHE PATENTSCHRIFT

(11) **EP 2 121 076 B1**
(45) Veröffentlichungstag und Bekanntmachung des Hinweises auf die Patenterteilung: **05.04.2017**
(21) Anmeldenummer: 07858231.9
(22) Anmeldetag: 28.12.2007
(51) Int. Cl.: A61M 39/02, A61M 5/14, A61M 5/142

(54) **EXTRAKORPORAL TRAGBARE INFUSIONSVORRICHTUNG**
INFUSION DEVICE THAT CAN BE WORN OUTSIDE THE BODY
DISPOSITIF DE PERFUSION PORTABLE EXTRACORPOREL

(30) Priorität: 29.12.2006 CH 21262006
(43) Veröffentlichungstag der Anmeldung: 25.11.2009
(73) Patentinhaber: F. Hoffmann-La Roche AG, 4002 Basel (CH); Roche Diabetes Care GmbH, 68305 Mannheim (DE)
(72) Erfinder: DE POLO, Marco, San Mate, California 94403 (US)
(74) Vertreter: Rentsch Partner AG
(86) Internationale Anmeldenummer: PCT/EP2007/064645
(87) Internationale Veröffentlichungsnummer: WO 2008/080990

(56) Entgegenhaltungen:
- EP-A2- 1 682 203
- WO-A-2006/120253
- WO-A1-2004/110526
- DE-A1- 10 255 817
- US-A- 5 472 317
- US-A1- 2003 088 238
- US-A1- 2003 153 900
- US-A1- 2004 059 316
- US-A1- 2005 171 512
- US-A1- 2006 264 890

## Beschreibung

Die Erfindung betrifft eine Vorrichtung zum automatisierten Abgeben eines flüssigen Medikaments gemäss dem Oberbegriff von Patentanspruch 1.

Vorrichtungen zum automatisierten und kontinuierlichen Abgeben von flüssigen Medikamenten kommen bevorzugterweise bei Patienten zur Anwendung, welche einen kontinuierlichen und über den Tag variierenden Bedarf an einem ausschliesslich subkutan verabreichbaren Medikament haben. Konkrete Anwendungsfälle sind z. B. bestimmte Schmerztherapien und die Behandlung von Diabetes mellitus, bei denen am Körper tragbare computergesteuerte Verabreichungsvorrichtungen zum Einsatz kommen. Dabei besteht das grundsätzliche Bedürfnis nach einem geringen Gewicht und minimalen Gerätevolumen der Verabreichungsvorrichtung, um eine bequeme und diskrete Trageweise zu ermöglichen.

Im Stand der Technik sind konventionelle, extrakorporale Infusionsvorrichtungen für die therapeutische Verwendung gebräuchlich, welche mittels eines Tragesystemes am Körper oder in den Kleidern mitgeführt werden. Während des Schlafes des Patienten werden sie meistens auf einer festen Ablagestelle neben dem Bett abgelegt und bleiben über eine Katheterzuführung mit dem Körper in Verbindung, dabei wird die Fluidverabreichung aufrechterhalten.

Seit kurzer Zeit sind miniaturisierte Infusionsvorrichtungen, welche direkt an der Hautoberfläche befestigt werden, erhältlich. Das Anbringen der Verabreichungsvorrichtung an die Hautoberfläche und das Einbringen der Verabreichungskanüle in das Körpergewebe wird vom Patienten eigenhändig vorgenommen. Nachdem eine solche Infusionsvorrichtung an der Hautoberfläche befestigt worden ist, klebt sie in der Regel 4 bis 5 Tage am Körper und wird danach ganz oder teilweise entsorgt.

Sowohl die an der Hautoberfläche befestigten als auch die konventionellen Infusionsvorrichtungen weisen typenspezifische und situationsbedingte Vor- und Nachteile auf. So ist es beispielsweise im Alltagsgebrauch des Patienten wünschenswert, dass die Infusionsvorrichtung, zumindest teilweise, temporär entfernbar ist, um beispielsweise ungehindert einer sportlichen Betätigung nachzugehen oder um Präzisionsteile oder Elektronikkomponenten der Verabreichungsvorrichtung bei einem Dusch- oder Badevorgang nicht unerwünschtem Wasserkontakt auszusetzen.

Mit einer konventionellen Verabreichungsvorrichtung ist durch eine temporäre Dekonnektierung des Katheterschlauches, mit einer geeigneten Abdichtung der Koppelstellen, eine zeitweilige Entfernung möglich. Ebenso ist der Ersatz oder Austausch von Hilfsmittel für die Einmalverwendung, z.B. Insulinampullen, Batterien, Katheter bei den konventionellen Verabreichungsvorrichtungen problemlos durchführbar, da die Vorrichtungen für eine lange Lebensdauer ausgelegt sind.

Demgegenüber erlauben die an der Hautoberfläche getragenen Verabreichungsvorrichtungen, aufgrund des konsequent auf Einmalverwendung und geringen Herstellungskosten ausgerichteten Konzeptes, weder eine temporäre Dekonnektierung noch einen Austausch von Teilen der Verabreichungsvorrichtung. Für körperliche Aktivitäten wie z. B. Sport, Schwimmsport oder Körperreinigung ist eine patientengerechte, temporäre Dekonnektierung vom Körper nicht möglich. Selbst bei starker Miniaturisierung von an der Hautoberfläche getragenen Verabreichungsvorrichtungen bleibt der Patient in der Bewegungsfreiheit eingeschränkt, da immer noch ein gewisses Bauteilvolumen am Körper angeordnet ist und eine statische Unwucht den Tragekomfort einschränkt. Des weiteren besteht die Gefahr, dass sich das Gerät durch ungenügende Haftung am Körper, beispielsweise bei Befestigung mittels eines Pflasters, vom Körper löst oder durch ruckartige Bewegungen vom Körper gerissen wird. Ferner sind die Injektionsstellen und die Tragstellen für dieses Konzept limitiert. Als Vorteile von an der Hautoberfläche getragenen Verabreichungsvorrichtungen sind eine unauffällige, diskrete Trageweise und kurze Fluidverbindungen anzuführen.

In der EP 1527792 A1 ist eine auf der Hautoberfläche befestigbare Verabreichungsvorrichtung beschrieben, welche jedoch nicht temporär dekonnektierbar ist und weitgehend dem Oberbegriff von Anspruch 1 entspricht.

Die US 2003/0088238 A1 betrifft ein modular aufgebautes tragbares medizinisches Gerät zur dosierten Verabreichung eines flüssigen Medikaments, mit wenigstens einer Reservoireinheit, einer Fördereinheit zur dosierten Abgabe des Medikaments aus dem Reservoir und einer Kontrolleinheit zur Steuerung der Fördereinheit. Die einzelnen Module können zu einem Gerät zusammengefügt und mit einer Infusionsnadel bestückt oder verbunden werden. Das Gerät und/oder die Injektionsnadel bzw. ein Injektionsnadelhalter können an der Unterseite mit einer klebenden Schicht versehen werden, die ein Aufkleben des Geräts oder des Nadelhalters auf der Haut des Patienten ermöglicht.

Die DE 102 55 817 A1 betrifft einen Kathederkopf mit verschließbarem Dichteelement. Der Kathederkopf weist einen Grundkörper auf mit einem Fluidkanal und einer vom Grundkörper abragende Kanüle zum Einführen in ein Gewebe. Der Kathederkopf weist weiterhin einen Verbindungskörper auf, der abnehmbar mit dem Grundkörper verbunden ist. Ein vom Gehäuse des Grundkörpers gelagertes Verschlusselement dichtet den Grundkörper ab, wenn der Verbindungskörper nicht mit dem Grundkörper verbunden ist.

Die US 5,472,317 A betrifft einen Befestigungsklipp für eine Infusionspumpe, mit dem die Infusionspumpe an einem Gürtel oder einem Kleidungsstück des Patienten wieder abnehmbar befestigt werden kann.

Es ist eine Aufgabe der Erfindung, ein tragbares medizinisches Gerät zur dosierten Verabreichung eines flüssigen Medikamentes zu schaffen, welches wahlweise direkt an der Körperoberfläche getragen und auch in konventioneller Trageweise vom Patienten mitgeführt werden kann, je nachdem, was in der jeweiligen Situation gewünscht wird.

Die Aufgabe wird durch den Gegenstand von Anspruch 1 gelöst. Vorteilhafte Ausführungsformen ergeben sich aus den Unteransprüchen.

Die Erfindung geht von einer extrakorporal tragbaren Infusionsvorrichtung aus, welche eine Reservoireinheit mit einem Reservoir für ein flüssiges Medikament und eine Fördereinheit zur dosierten Abgabe des Medikamentes aufweist. Die Fördereinheit umfasst geeignete Mittel zur Förderung und dosierten Ausschüttung des Medikaments.

Die Erfindung ist nicht auf die Verabreichung von Insulin beschränkt, sondern erstreckt sich generell auf die Verabreichung von Produkten, die per Infusion verabreichbar sind. Vergleichbare Geräte können nicht nur in der Selbstverabreichung, sondern auch im stationären Bereich, beispielsweise unter ständiger ärztlicher Aufsicht in Kliniken, beispielsweise in der Rehabilitation oder sonstigen Nachbehandlung, zum Einsatz kommen, wo sie vom Patienten getragen werden.

In einer bevorzugten Ausführungsform der Verabreichungsvorrichtung ist die Vorrichtung derart ausgebildet, dass die Fördereinheit und die Reservoireinheit der tragbaren Verabreichungsvorrichtung mittels Ankopplungsmitteln mechanisch und funktional zusammenfügbar bzw. separierbar sind. Dabei werden die Fördereinheit, Reservoireinheit und ein Adapter, der eine bevorzugt flexible oder gegebenenfalls starre Adapterplatte für eine Auflage an der Kleidung oder direkt am Körper umfassen kann, zu einer integrierten Gesamteinheit vereinigt.

Die WO2004/110526A1 offenbart eine tragbare modulare Infusionspumpe, bei der ein Pumpengehäuse, welches seinerseits ein Produktreservoir und eine Fördereinheit aufnimmt, unmittelbar am Infusionsort auf der Haut des Patienten angeordnet wird, wobei eine Infusionskanüle von der Unterseite des Pumpengehäuses abragt. Steuergerät für die Fördereinrichtung ist in einem separaten Gehäuse angeordnet und wird im Betrieb von dem Patienten räumlich getrennt mitgeführt bzw. getragen.

Die US20030153900A1 offenbart ein in seiner Gesamtheit am Körper getragene Abgabevorrichtung für Medikamente, wobei eine von der Unterseite der Vorrichtung abragende Kanüle in die Haut eindringt. Die Vorrichtung kann entweder mittels Klebstoff auf der Haut oder alternativ an einem integrierten Clip an einem Kleidungsstück (Unterwäsche) befestigt werden.

Die WO2005/039673A2 offenbart eine modulare Infusionspumpe mit einer Steuereinheit und einer mit der Steuereinheit zu einer kompakten Einheit verbindbaren Reservoireinheit, wobei die resultierende Einheit zur Anwendung unmittelbar auf die Haut geklebt wird. Die Infusion erfolgt wahlweise über eine von einer Unterseite abgragende Kanüle oder eine mittels Katheter bzw. Infusionsleitung verbundene Kanüle

In bevorzugten Ausführungen ist ein oder sind mehrere Ankopplungsmittel an der Fördereinheit und ein oder mehrere Ankopplungsmittel an der Reservoireinheit, bevorzugt jeweils ohne Werkzeug nicht entfernbar ausgebildet, z. B. indem sie einstückig mit dem jeweiligen Gehäuse geformt oder stoffschlüssig verbunden oder anders fest gefügt, beispielsweise verschraubt sind. Hierdurch gibt sich der Vorteil, dass die Ankopplungsmittel unverlierbar mit der jeweils zugeordneten der beiden Einheiten geformt oder verbunden sind und damit jederzeit zur Verfügung stehen.

In einer bevorzugten Ausführungsform werden die genannten Einheiten der Verabreichungsvorrichtung (Pumpe), Reservoir- und Fördereinheit, an einen fluidisch-mechanischen Konnektor des Adapters angekoppelt, welcher sowohl die Einheiten relativ zum Adapter, vorzugweise der Adapterplatte, fixiert als auch eine fluidische Verbindung zwischen Reservoireinheit und einer den Körper infundierenden Kanüle für eine bevorzugt subkutane Verabreichung gewährleistet. Der Konnektor ist vorteilhafterweise in der Mitte der Adapterplatte angeordnet. Die mechanische Kopplung an den Konnektor kann kraftschlüssig durch Klemmung an den Konnektor oder formschlüssig durch Bildung einer Gegenkontur durch die konnektierten Gehäuseteile der Reservoir- und Fördereinheit erfolgen.

In einer weiteren bevorzugten Ausführungsform der Vorrichtung sind die Ankopplungsmittel derart ausgestaltet, dass das Zusammenfügen von Reservoireinheit und Fördereinheit durch ein Bewegen derselben relativ zueinander beispielsweise entlang von starren Führungen erfolgen kann, insbesondere durch Zusammenschieben entlang von linearen Führungen, wie beispielsweise lineare Schwalbenschwanzführungen, oder durch einen von beiden Einheiten gebildeten Bajonettverschluss. Eine derartige Art der Ankopplung ermöglicht eine exakte Positionierung der beiden Einheiten zueinander und erleichtert zudem insbesondere ein eigenhändiges Zusammenfügen von Reservoireinheit und Fördereinheit an unzugänglichen oder schwer einsehbaren Stellen, z. B. bei seitlich am Gürtel befestigter Adapterplatte.

Bevorzugt sind die Ankoppelungsmittel derart ausgestaltet, dass das Zusammenfügen von Fördereinheit und Reservoireinheit durch Einrasten von Rastelementen erfolgen kann. Solche Ankopplungsmittel kommen in der Regel ohne zusätzliche Sicherungsmittel aus.

In noch einer weiteren bevorzugten Ausführungsform sind die Ankoppelungsmittel derart ausgestaltet, dass das Zusammenfügen von Reservoireinheit und Fördereinheit kraftschlüssig, bevorzugterweise durch Klemmung, oder temporäre Klebung, Magnetkraft oder über Klettpaarung erfolgt. Ebenfalls können die Ankopplungsmittel derartig ausgestaltet sein, dass das Zusammenfügen von Reservoireinheit und Fördereinheit unter Dehnung eines oder mehreren von der Einheit gebildeten gummielastischer Formelemente erfolgt, welche(s) im zusammengefügten Zustand durch Umschliessen von oder Eingreifen in eine oder mehrere Gegenkonturen einen elastisch vorgespannten Formschluss mit zugeordneten, bevorzugt starren Formelementen der komplementären Einheit bilden. Hierdurch lässt sich auf eine kostengünstige und einfache Weise eine spielfreie Ankopplung realisieren.

In einer besonders bevorzugten Ausführungsform werden die Einheiten mittels eines Adapters mit bevorzugt einer Adapterplatte, welche während der temporären Dekonnektierung auf der Hautoberfläche verbleibt und ein sogenanntes Verweilteil bildet, über eine lösbare Klebeverbindung oder einem Hautpflaster an der Hautoberfläche befestigt. Diese Ausführungsform vereinigt die Vorteile der diskreten Tragemöglichkeit der körperbefestigten extrakorporalen Infusionsvorrichtungen mit der patientenfreundlichen Möglichkeit einer temporären Dekonnektierung.
In einer besonders bevorzugten alternativen Ausführungsform werden die Pumpenteile über einen Adapter mit bevorzugt einer Adapterplatte zur Mitführung an einem Tragesystem des Patienten angekoppelt. Eine solche Adapterplatte ist derart ausgestaltet ist, dass Verbindungsmittelmittel zu einem Tragesystem, insbesondere eine Umhängeschlaufe, eine körperumfassende Gurtschlaufe, einen Gürtel-Clip oder einer Befestigungsmöglichkeit an einem Kleidungsstück, vorhanden sind. Hierdurch wird es auf einfache Weise möglich, die Infusionsvorrichtung durch Austausch des Tragesystemes an verschiedene Alltagssituationen bzw. Verwendungen anzupassen. Die Vorrichtung mit den Pumpenteilen und der Adapterplatte kann als Ganzes an einem Gürtel getragen werden. Zu diesem Zweck ist ein seitlicher Kanülenanschluss an der Adapterplatte notwendig. An der somit entstehenden planen Unterseite der Adapterplatte können ein oder mehrere Befestigungsclips oder eine Gurtschlaufe angebracht sein.

In einer besonders bevorzugten Ausführungsform bei Verwendung einer Adapterplatte zur Mitführung an ein Tragesystem, ist ein dekonnektierbarer Katheterkopf, mit einer Kanüle zur Einführung in das Körpergewebe, über eine kurze Katheterleitung mit der Adapterplatte verbunden Dekonnektierbare Katheterköpfe sind im Stand der Technik bekannt und werden standardmässig in der Pumpentherapie verwendet. In einer derartigen Ausführungsform erübrigt sich ein Septum am fluidisch-mechanischen Konnektor der Adapterplatte.

In einer noch weiteren besonders bevorzugten Ausführungsform der Vorrichtung umfassen die Ankoppelungsmittel Sicherungsmittel, mittels welchen die Reservoireinheit und die Fördereinheit in bestimmungsgemäss zusammengefügtem Zustand bevorzugterweise gegenüber unbeabsichtigtes Separieren gesichert werden können. Dabei ist es bevorzugt, wenn die Sicherungsmittel zur Aufhebung der Sicherung eine Manipulation erfordern, welche verschieden ist von der zur Separierung erforderlichen Manipulation, also beispielsweise ein Pressen eines Entriegelungsknopfes erfordern, wenn für das Separieren eine Drehbewegung erforderlich ist. Hierdurch kann ein Höchstmass an Sicherheit gegen ein unbeabsichtigtes Separieren und Verlieren erzielt werden.

In einer weiteren besonders bevorzugten Ausführungsform sind bevorzugt elektronische Mittel auf der Vorrichtung vorgesehen, welche vor einer Separierung einen gesicherten Ruhezustand der Infusionsvorrichtung herbeiführen und nach dem Zusammenführen eine Weiterführung der programmgemässen Verabreichung ermöglichen.

In ebenfalls besonders bevorzugten Ausführungsformen besteht die Fördereinheit wahlweise aus einer Fördereinheit zur Einmalverwendung oder zur Mehrfachverwendung, die Reservoireinheit wahlweise aus Reservoireinheit zur Einmalverwendung oder zur Mehrfachverwendung und die Ausführungsformen der Adapterplatten wahlweise zur Einmalverwendung oder Mehrfachverwendung.

Weitere bevorzugte Ausführungen der Erfindung ergeben sich aus den abhängigen Ansprüchen und aus der nun folgenden Beschreibung anhand der Figuren. Dabei zeigen:
- Figur 1: eine Reservoireinheit, eine Fördereinheit und eine Adapterplatte zur direkten Befestigung an der Hautoberfläche;
- Figur 2: eine Adapterplatte zum Mitführen an einem Tragesystem in Form eines körperumfassenden Gurtes, wobei ein kurzes Katheterschlauchstück an der Adapterplatte angebracht ist, welches die Distanz zwischen Trageposition und Kanüleneinstichposition überwindet;
- Figur 3: eine Adapterplatte zur direkten Befestigung an der Hautoberfläche, wobei die Fluidzuführung in der Adapterplatte verläuft;
- Figur 4: eine Adapterplatte zum Mitführen an einem Tragesystem, wobei die Fluidzuführung in der Adapterplatte verläuft.

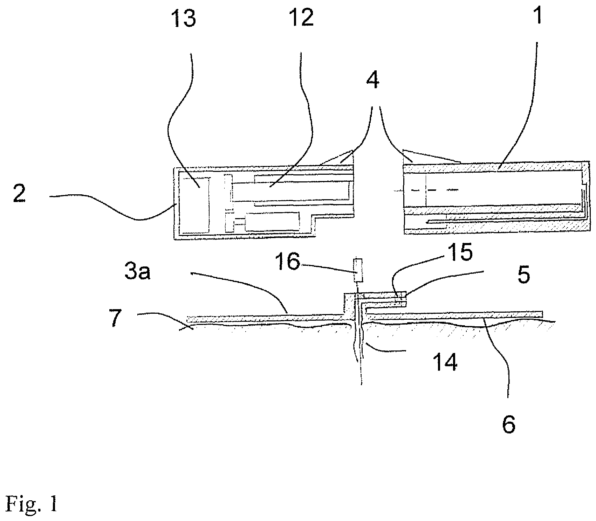

Figur 1 zeigt eine erfindungsgemässe Infusionsvorrichtung mit einer koppelbaren Fördereinheit (2) und einer Reservoireinheit (1), welche mittels einer erfindungsgemäßen Adapterplatte (3a) an der Hautoberfläche (7) befestigt ist. Aufgrund einer vollständigen Befestigung der Infusionsvorrichtung an der Körperoberfläche können unter Umständen hohe Gewichts- und Beschleunigungskräfte entstehen, dementsprechend muss ein stark haftendes Pflaster (6) gewählt werden, welches gut haftet aber gleichzeitig einfach zu entfernen ist.

Die Nachteile eines permanent am Körper befestigten Gerätes können damit gelöst werden, dass die Reservoireinheit (1) und die Fördereinheit (2), welche die Antriebseinheit (12) und die Elektronik (13) enthält, über einen Adapter am Körper befestigt werden. Der Adapter weist eine auf der Haut aufliegende Adapterplatte (3a) und einen von dieser abragenden fluidisch-mechanischen Konnektor (5) auf. Die Einheiten (1) und (2) sind über den Konnektor (5) mittels einfacher Handgriffe von der Adapterplatte (3a) lösbar, während diese am Körper verbleiben kann, und somit temporär vom Körper entfernbar, um beispielsweise ungehindert einer sportlichen Betätigung nachzugehen oder um Präzisionsteile oder Elektronikkomponenten der Verabreichungsvorrichtung bei einem Dusch- oder Badevorgang nicht unerwünschtem Wasserkontakt auszusetzen. Die Verbindung von Fördereinheit (2), Reservoireinheit (1) und Adapter (3a, 5) erfolgt durch Ankopplungsmittel (4), welche durch Schnappen, Schrauben oder durch einen Schnellverschluss (z.B. Hebel und Gurte, Schnalle) wirken. Der Adapter (3a, 5), mit einer integrierten Kanüle (14) zum Einbringen in das Körpergewebe (7), dient als Verbindung- und Befestigungssystem der Insulinpumpe und verbindet die Fördereinheit (2) mit der Reservoireinheit (1). Ein Septum (15) verhindert während einer temporären Dekonnektierung den Austritt von Körperflüssigkeit aus dem Körper. Eine auf dem fluidisch-mechanischen Konnektor (5) einbringbare Einstechnadel (16) erlaubt eine Penetration des Körpergewebes (7) beim Applizieren der Kanüle (14). Die Reservoireinheit kann dabei als tragender Gehäuseteil verwendet werden mit dem Vorteil, dass die ganze Geräteeinheit sehr kompakt und flach konstruiert werden kann. Ein zusätzliches Ampullenfach, wie es bei den herkömmlichen Insulinpumpen verwendet wird, entfällt bei dieser Anordnung. Die Handhabung wird ebenfalls vereinfach, da kein zusätzlicher Adapter notwendig ist, um die Reservoireinheit (1) und die Fördereinheit (2) mechanisch und erstere auch fluidisch mit der Kanüle (14) zu verbinden. Die Reservoireinheit (1) ist dabei so ausgelegt, dass durch vorhandene Verstärkungen (z.B. Rippen) keine Zusatzförderung infolge äusserer Kräfte auftreten kann.

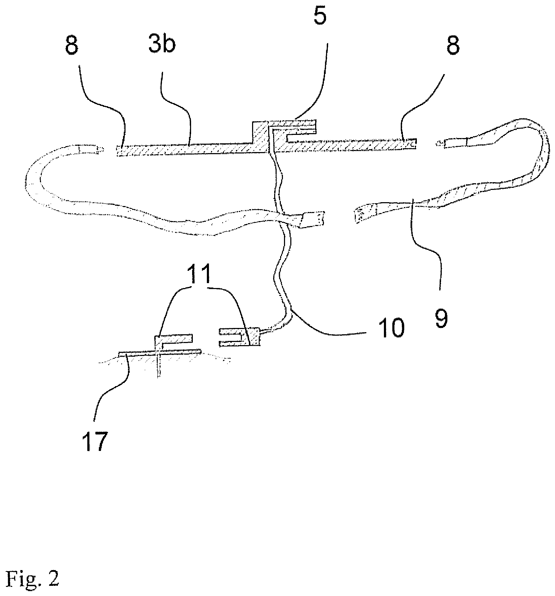

Figur 2 zeigt eine Adapterplatte (3b) für eine Verabreichungsvorrichtung, welche über ein Tragesystem am Körper des Patienten mitgeführt wird. Als Alternative zu einer körperbefestigten Trageweise mittels Adapterplatte (3a) können die Reservoireinheit (1) und die Fördereinheit (2) mit der Adapterplatte (3b) kombiniert werden, welche über einen kurzen (<50 cm) Katheter (10) die fluidische Verbindung zum Katheterkopf (11) ermöglicht und mittels Gurttragsystem (8, 9) vom Patienten am Körper mitgeführt werden kann. Durch die vorwiegende Aufnahme der Kräfte über das Tragesystem ist es möglich, ein kleineres Pflaster (16) für die Kanülenbefestigung zu verwenden, womit die Gefahr der Hautirritation herabgesetzt wird und Befestigungsprobleme reduziert werden können. Um ein temporäres Ablegen der Infusionsvorrichtung zu ermöglichen, ist der Katheterkopf dekonnektierbar ausgeführt. Eine Entkoppelung der Reservoireinheit (1) und der Fördereinheit (2) ist in diesem Falle nicht zwingend notwendig. In einem solchen Fall erübrigt sich auch das Septum im Konnektor.

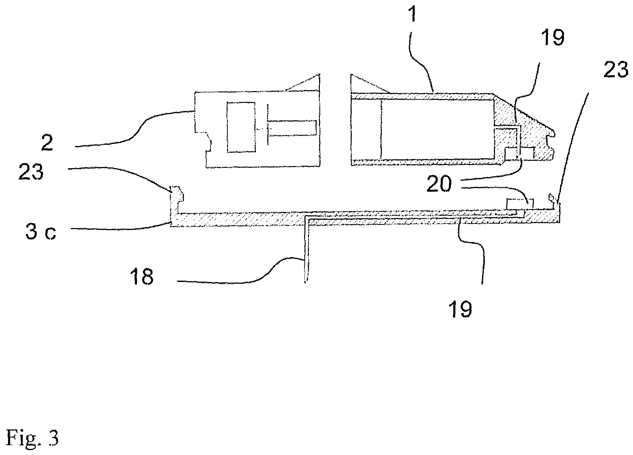

Figur 3 zeigt eine weitere Ausführungsform einer Adapterplatte (3c) zur direkten Befestigung an der Hautoberfläche, bei welcher eine Katheterzuleitung (19) stromabwärts der Reservoireinheit (1) in die Adapterplatte integriert ist und direkt an der Hautoberfläche getragen werden kann. Die Kanüle (18) ist in der Mitte der Adapterplatte (3c) angeordnet und von symmetrisch angeordnetem Hautpflaster oder Klebemittel umgeben. Diese Anordnung bewirkt, dass die Kräfte, welche auf die Injektionsstelle einwirken allseitig gleich sind und somit der Tragkomfort erhöht wird. Die fluidische Konnektierung zwischen der Reservoireinheit (1) und der Adaptereinheit (3c) erfolgt mit einem Septum/Nadelanschluss (20). Die mechanische Konnektierung erfolgt über jeweils stirnseitig angebrachte mechanische Kopplungsmittel (23).

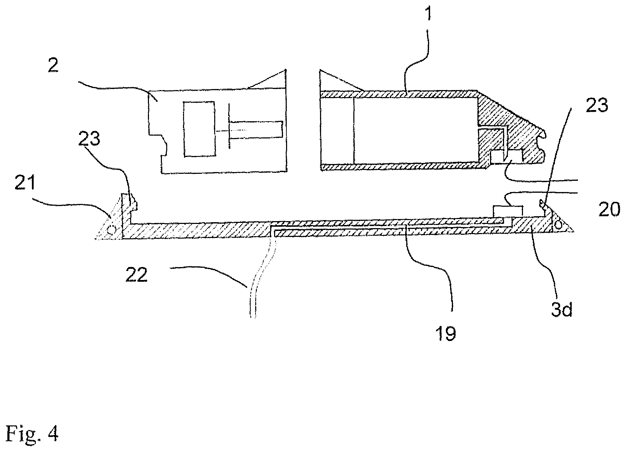

Figur 4 zeigt eine Ausführungsform einer Adapterplatte (3d), welche zum Mitführen an einem Tragesystem vorgesehen ist. Die fluidische Konnektierung zwischen der Reservoireinheit und der Adaptereinheit (3d) erfolgt über einen Septum/Nadelanschluss (20), die anschliessende Katheterzuleitung (19) ist in die Adapterplatte integriert. Die mechanische Konnektierung erfolgt über jeweils stirnseitig angebrachte mechanische Kopplungsmittel (23). Die stromabwärts führende Katheterzuleitung (22) ist, fix oder lösbar, an Adapterplatte angebracht. In einer nicht gezeigten Darstellung kann die Katheterzuleitung auch seitlich der Adapterplatte wegführen, um beispielsweise eine Befestigung an einem Gurt zu ermöglichen. An den stirnseitigen Enden an der Adapterplatte (3d) sind Befestigungsmöglichkeiten (20) für ein Tragesystem angebracht.

### Bezugszeichenliste:

- 1: Reservoireinheit
- 2: Fördereinheit
- 3a: Adapterplatte zur direkten Befestigung an der Hautoberfläche
- 3b: Adapterplatte für ein Tragesystem
- 3c: Adapterplatte zur direkten Befestigung an der Hautoberfläche
- 3d: Adapterplatte für ein Tragesystem
- 4: Ankopplungsmittel (Reservoireinheit/Fördereinheit)
- 5: Fluidisch-mechanischer Konnektor
- 6: lösbare Klebeverbindung oder Hautpflaster
- 7: Hautgewebe
- 8: Verbindungsmittel zu einem Tragesystem
- 9: körperumfassende Gurtschlaufe
- 10: Katheterleitung
- 11: dekonnektierbarer Katheterkopf mit Einstechkanüle
- 12: Antriebseinheit für die Fördereinheit
- 13: Steuereinheit
- 14: Kanüle
- 15: Septum
- 16: Einstechnadel zum Einbringen der Kanüle in das Körpergewebe
- 17: Hautpflaster
- 18: in Adapterplatte integrierte Kanüle
- 19: Kanülenzuleitung, in Adapterplatte integriert
- 20: Septum /Nadel-Verbindung
- 21: Befestigungsmittel für eine Verbindung mit einem Tragesystem
- 22: Katheterleitung zu einem Katheterkopf mit Kanüle
- 23: mechanische Kopplungsmittel zur Aufnahme von Förder-/Reservoireinheit

## Patentansprüche

1. Tragbares medizinisches Gerät zur dosierten Verabreichung eines flüssigen Medikamentes, welches umfasst:
a) ein erstes Gehäuse mit einer Reservoireinheit (1) mit einem Auslass in eine stromabwärts der Reservoireinheit (1) gelegene Fluidstrecke zur Durchleitung des Medikamentes,
b) ein zweites Gehäuse mit einer Fördereinheit (2) zur dosierten Abgabe des Medikaments aus der Reservoireinheit (1) und geeigneten Mitteln zur Steuerung der Fördereinheit (2),
c) einen Adapter (3a, 3c,) mit einer Adapterplatte und mit Ankopplungsmitteln (5; 20,23) zur Aufnahme des ersten Gehäuses mit der Reservoireinheit (1) und des zweiten Gehäuses mit der Fördereinheit (2),
d) und Befestigungsmitteln (6) in Form einer lösbaren Klebeverbindung oder eines Hautpflasters zur extrakorporalen Mitführung des Adapters (3a, 3c) am Patienten durch direkte Befestigung an der Hautoperfläche des Patienten mit einer Auflage der Adapterplatte des Adapters (3a, 3c) direkt an der Hautoberfläche,
c') einen weiteren Adapter (3b, 3d) mit einer Adapterplatte und mit Ankoppelmitteln (5, 20,23) zur Aufnahme des ersten Gehäuses mit der Reservoireinheit (1) und des zweiten Gehäuses mit der Fördereinheit (2),
d') und Befestigungsmitteln (8, 21) in Form von Verbindungsmitteln (8, 21) zu einem Tragesystem (9) zur extrakorporalen Mitführung des weiteren Adapters (3b, 3d) am Patienten durch Tragen auf einem Kleidungsstück mit Auflage der Adapterplatte des weitere Adapters (3b, 3d) auf dem Kleidungsstück,
d") wobei der weitere Adapter (3b, 3d), vorzugsweise die Adapterplatte (3b, 3d) des weiteren Adapters, über eine Katheterleitung (10) mit einem dekonnektierbaren Katheterkopf (11) zur Infusion des Medikamentes verbunden ist,
e) wobei die Fördereinheit (2) und die Reservoireinheit (1) zu einer mechanischen und fluidischen Gesamteinheit zusammenfügbar sind, vorzugsweise zumindest weitgehend werkzeuglos,
f) und wahlweise an den Adapter (3a, 3c) oder den weiteren Adapter (3b, 3d) ankoppelbar und von dem Adapter (3a, 3c) bzw. dem weiteren Adapter (3b, 3d) abkoppelbar und dadurch wahlweise mit dem Adapter (3a, 3c) oder dem weiteren Adapter (3b, 3d) zu einer mechanischen und fluidischen Gesamteinheit zusammenfügbar sind, vorzugsweise zumindest weitgehend werkzeuglos,
h) wobei der Adapter (3a, 3c) und der weitere Adapter (3b, 3d) jeweils eine Fluidverbindung zwischen der Reservoireinheit (1) und einer den Körper infundierenden Injektionsnadel (18) oder Kanüle (14 ) bereit stellt und einen mit der jeweiligen Adapterplatte geformten oder gefügten Konnektor (5) für eine mechanische und fluidische Verbindung zwischen der Reservoireinheit (1) und der Fördereinheit (2) einerseits und dem jeweiligen Adapter (3a, 3b, 3c, 3d) andererseits aufweist.

2. Tragbares medizinisches Gerät gemäss Anspruch 1, **dadurch gekennzeichnet, dass** Ankopplungsmittel (4), welche eine mechanische und funktionale Koppelung zwischen der Fördereinheit (2) und der Reservoireinheit (1) ermöglichen, zumindest weitgehend auf der Fördereinheit (2) und der Reservoireinheit (1) ausgebildet sind.

3. Tragbares medizinisches Gerät gemäss einem der vorangehenden Ansprüche, **dadurch gekennzeichnet, dass** die Fördereinheit (2), die Reservoireinheit (1) und der jeweilige Adapter (3a, 3b, 3c, 3d) über eine zumindest weitgehend werkzeuglos herstellbare kraftschlüssige oder formschlüssige Verbindung untereinander verbindbar sind.

4. Tragbares medizinisches Gerät gemäss einem der vorangehenden Ansprüche, **dadurch gekennzeichnet, dass** die Fördereinheit (2) und die Reservoireinheit (1) an einem fluidischen-mechanischen Konnektor (5) des jeweiligen Adapters (3a, 3b, 3c, 3d) befestigbar und fluidisch mit einem Auslass des jeweiligen Adapters (3a, 3b, 3c, 3d) verbindbar sind.

5. Tragbares medizinisches Gerät gemäss einem der vorangehenden Ansprüche, **dadurch gekennzeichnet, dass** die Fördereinheit (2) und die Reservoireinheit (1) an einem fluidischen-mechanischen Konnektor (5) des jeweiligen Adapters formschlüssig oder kraftschlüssig zusammenfügbar sind.

6. Tragbares medizinisches Gerät gemäss einem der Ansprüche 1 bis 4, **dadurch gekennzeichnet, dass** entweder die Fördereinheit (2) oder die Reservoireinheit (1) mit einem fluidischen-mechanischen Konnektor (5) des Adapters kraftschlüssig oder formschlüssig zusammenfügbar ist.

7. Tragbares medizinisches Gerät gemäss einem der Ansprüche 1 bis 6, **dadurch gekennzeichnet, dass** die Verbindungsmittelmittel (8) des weiteren Adapters (3b, 3d) Verbindungsmittel zu einem Tragesystem in Form einer Umhängeschlaufe, einer körperumfassenden Gurtschlaufe (9), einem Gürtel-Clip oder einer Befestigungsmöglichkeit an einem Kleidungsstück sind.

8. Tragbares medizinisches Gerät gemäss dem vorhergehenden Anspruch, **dadurch gekennzeichnet, dass** die Verbindungsmittel (8) mit Sicherungsmitteln gegen unbeabsichtigtes Separieren vom Tragesystem ausgestattet sind.

9. Tragbares medizinisches Gerät gemäss einem der vorangehenden Ansprüche, **dadurch gekennzeichnet, dass** elektronische Mittel vorgesehen sind, welche vor einer Separierung einen gesicherten Ruhezustand der Infusionsvorrichtung herbeiführen können, vorzugsweise automatisch durch die Separierung ausgelöst, und nach dem Zusammenführen eine Weiterführung einer programmgemässen Verabreichung ermöglichen, vorzugsweise automatisch durch die Zusammenführung ausgelöst.

10. Tragbares medizinisches Gerät gemäss einem der vorangehenden Ansprüche, **dadurch gekennzeichnet, dass** die Fördereinheit (2) wahlweise aus einer Fördereinheit zur Einmalverwendung oder zur Mehrfachverwendung besteht.

11. Tragbares medizinisches Gerät gemäss einem der vorhergehenden Ansprüche, **dadurch gekennzeichnet, dass** die Reservoireinheit (1) zur Einmalverwendung vorgesehen ist.

12. Tragbares medizinisches Gerät gemäss einem der vorangehenden Ansprüche, **dadurch gekennzeichnet, dass** zumindest der auf der Haut tragbare Adapter (3a, 3c) nur zur Einmalverwendung vorgesehen ist.

## Claims

1. A portable medical device for the dosed administration of a liquid medication, said device comprising:
a) a first housing with a reservoir unit (1) having an outlet into a fluid path for conducting the medication, which is downstream of said reservoir unit (1),
b) a second housing with a conveying unit (2) for the dosed dispensing of the medication from the reservoir unit (1) and suitable means for controlling said conveying unit (2),
c) an adapter (3a, 3c) with an adapter plate and with coupling means (5; 20, 23) for receiving the first housing with the reservoir unit (1) and the second housing with the conveying unit (2),
d) and attachment means (6) in the form of a detachable adhesive bond or a skin patch so that the adapter (3a, 3c) can be carried extracorporeally on the patient by direct attachment to the surface of the patient's skin by placing the adapter plate of the adapter (3a, 3c) directly on the skin surface,
c') a further adapter (3b, 3d) having an adapter plate and coupling means (5; 20, 23) for receiving the first housing with the reservoir unit (1) and the second housing with the conveying unit (2),
d') and attachment means (8, 21) in the form of connecting means (8, 21) to a carrying system (9) for carrying the further adapter (3b, 3d) extracorporeally on the patient such that it is worn on an article of clothing with the adapter plate of the further adapter (3b, 3d) placed on the article of clothing,
d") wherein the further adapter (3b, 3d), preferably the adapter plate (3b, 3d) of the further adapter, is connected to a disconnectable catheter head (11) via a catheter line (10) for the infusion of the medication,
e) wherein the conveying unit (2) and the reservoir unit (1) can be combined into a complete mechanical and fluidic unit, preferably at least substantially without the use of tools,
f) and can optionally be coupled with the adapter (3a, 3c) or the further adapter (3b, 3d) and decoupled from the adapter (3a, 3c) or the further adapter (3b, 3d) and thereby can optionally be combined with the adapter (3a, 3c) or the further adapter (3b, 3d) into a complete mechanical and fluidic unit, preferably at least substantially without the use of tools,
h) wherein the adapter (3a, 3c) and the further adapter (3b, 3d) each establishes a fluid connection between the reservoir unit (1) and an injection needle (18) or cannula (14) that infuses the drug the body, and each has a connector (5) that is molded or fused to the respective adaptor plate for a mechanical and fluidic connection between the reservoir unit (1) and the conveying unit (2) at one end and the respective adapter (3a, 3b, 3c, 3d) at the other end.

2. The portable medical device according to claim 1, **characterized in that** coupling means (4), which permit a mechanical and functional coupling between the conveying unit (2) and the reservoir unit (1), are arranged at least substantially on the conveying unit (2) and the reservoir unit (1),

3. The portable medical device according to one of the preceding claims, **characterized in that** the conveying unit (2), the reservoir unit (1) and the respective adapters (3a, 3b, 3c, 3d) can be interconnected by a force-fitting or form-fitting connection that can be established substantially without the use of tools.

4. The portable medical device according to one of the preceding claims, **characterized in that** the conveying unit (2) and the reservoir unit (1) can be attached to a fluidic-mechanical connector (5) of each respective adapter (3a, 3b, 3c, 3d) and can be fluidically connected to an outlet of each respective adapter (3a, 3b, 3c, 3d).

5. The portable medical device according to one of the preceding claims, **characterized in that** the conveying unit (2) and the reservoir unit (1) can be form-fittingly or force-fittingly joined to a fluidic-mechanical connector (5) of each respective adapter.

6. The portable medical device according to one of claims 1 through 4, **characterized in that** either the conveying unit (2) or the reservoir unit (1) can be form-fittingly or force-fittingly joined to a fluidic-mechanical connector (5) of the adapter.

7. The portable medical device according to one of claims 1 through 6, **characterized in that** the connecting means (8) of the further adapter (3b, 3d) are means for connecting to a carrying system in the form of a hanging strap, a belt strap (9) that surrounds the body, a belt clip or means for fastening to an article of clothing.

8. The portable medical device according to the previous claim, **characterized in that** the connecting means (8) are provided with means for securing it against unintended separation from the carrying system.

9. The portable medical device according to one of the preceding claims, **characterized in that** electronic means are provided which can bring about a secured resting state of the infusion device before a separation, preferably triggered automatically by the separation, and which can permit a programmed dosage to resume after reattachment, preferably triggered automatically by the fitting.

10. The portable medical device according to one of the preceding claims, **characterized in that** the conveying unit (2) optionally consists of a conveying unit for one-time use or for multiple usage.

11. The portable medical device according to one of the preceding claims, **characterized in that** the reservoir unit (1) is intended for one-time use.

12. The portable medical device according to one of the preceding claims, **characterized in that** at least the adapter (3a, 3c) that is worn on the skin is intended only for one-time use.

## Revendications

1. Appareil médical portatif pour l'administration dosée d'un médicament liquide, qui comprend :
a) un premier boîtier avec une unité de réservoir (1) ayant une sortie dans un trajet de fluide situé en aval de l'unité de réservoir (1) pour décharger le médicament,
b) un second boîtier avec une unité d'acheminement (2) pour la décharge dosée du médicament de l'unité de réservoir (1) et des moyens appropriés pour commander l'unité d'acheminement (2),
c) un adaptateur (3a, 3c) avec une plaque d'adaptateur et des moyens d'accouplement (5 ; 20, 23) pour recevoir le premier boîtier avec l'unité de réservoir (1) et le second boîtier avec l'unité d'acheminement (2),
d) et des moyens de fixation (6) sous la forme d'un joint collé enlevable ou d'un sparadrap pour le port extracorporel de l'adaptateur (3a, 3c) sur le patient par fixation directe à la surface de la peau du patient par application directe de la plaque d'adaptateur (3a, 3c) sur la surface de la peau,
(c') un autre adaptateur (3b, 3d) avec une plaque d'adaptateur et des moyens d'accouplement (5, 20, 23) pour recevoir le premier boîtier avec l'unité de réservoir (1) et le second boîtier avec l'unité d'acheminement (2),
d') et des moyens de fixation (8, 21) sous la forme de moyens de jonction (8, 21) à un système porteur (9) pour le port extracorporel de l'autre adaptateur (3b, 3d) sur le patient par support sur un article de vêtement avec application de la plaque d'adaptateur de l'autre adaptateur (3b, 3d) sur l'article de vêtement,
d") dans lequel l'autre adaptateur (3b, 3d), de préférence la plaque d'adaptateur (3b, 3d) de l'autre adaptateur, est raccordé(e) via une ligne de cathéter (10) à une tête de cathéter déconnectable (11) pour la perfusion du médicament,
e) dans lequel l'unité d'acheminement (2) et l'unité de réservoir (1) peuvent être jointes en une unité globale mécanique et fluidique, de préférence au moins largement sans outillage,
f) et peuvent au choix être accouplées à l'adaptateur (3a, 3c) ou à l'autre adaptateur (3b, 3d) et désaccouplées de l'adaptateur (3a, 3c) ou de l'autre adaptateur (3b, 3d) et, par suite, au choix jointes à l'adaptateur (3a, 3c) ou à l'autre adaptateur (3b, 3d) en une unité globale mécanique et fluidique, de préférence au moins largement sans outillage,
h) dans lequel l'adaptateur (3a, 3c) et l'autre adaptateur (3b, 3d) assurent respectivement une communication fluidique entre l'unité de réservoir (1) et une aiguille d'injection (18) ou une canule (14) en perfusion dans le corps et présentent un raccord (5) formé ou assemblé avec la plaque d'adaptateur respective pour assurer une communication mécanique et fluidique entre l'unité de réservoir (1) et l'unité d'acheminement (2), d'une part, et l'adaptateur respectif (3a, 3b, 3c, 3d), d'autre part.

2. Appareil médical portatif selon la revendication 1, **caractérisé en ce que** des moyens d'accouplement (4), qui permettent un accouplement mécanique fonctionnel entre l'unité d'acheminement (2) et l'unité de réservoir (1), sont formés au moins largement sur l'unité d'acheminement (2) et l'unité de réservoir (1).

3. Appareil médical portatif selon l'une quelconque des revendications précédentes, **caractérisé en ce que** l'unité d'acheminement (2), l'unité de réservoir (1) et l'adaptateur respectif (3a, 3b, 3c, 3d) peuvent être assemblés les uns aux autres via une jonction à force ou mécanique réalisable au moins largement sans outillage.

4. Appareil médical portatif selon l'une quelconque des revendications précédentes, **caractérisé en ce que** l'unité d'acheminement (2) et l'unité de réservoir (1) peuvent être fixés à un raccord mécanico-fluidique (5) à l'adaptateur respectif (3a, 3b, 3c, 3d) et raccordés au plan fluidique à une sortie de l'adaptateur respectif (3a, 3b, 3c, 3d).

5. Appareil médical portatif selon l'une quelconque des revendications précédentes, **caractérisé en ce que** l'unité d'acheminement (2) et l'unité de réservoir (1) peuvent être assemblés par voie mécanique ou à force à un raccord mécanico-fluidique (5) de l'adaptateur respectif.

6. Appareil médical portatif selon l'une quelconque des revendications 1 à 4, **caractérisé en ce que** l'unité d'acheminement (2) ou l'unité de réservoir (1) peut être assemblée à force ou par voie mécanique à un raccord mécanico-fluidique (5) de l'adaptateur.

7. Appareil médical portatif selon l'une quelconque des revendications 1 à 6, **caractérisé en ce que** les moyens de raccordement (8) de l'autre adaptateur (3b, 3d) sont des moyens de raccordement avec un système porteur sous la forme d'une boucle de suspension, d'une boucle de ceinture (9) entourant le corps, d'une pince de ceinture ou d'une possibilité de fixation sur un article de vêtement.

8. Appareil médical portatif selon l'une quelconque des revendications précédentes, **caractérisé en ce que** les moyens de raccordement (8) sont équipés de moyens de fixation pout s'opposer à une séparation involontaire du système porteur.

9. Appareil médical portatif selon l'une quelconque des revendications précédentes, **caractérisé en ce qu'**il est prévu des moyens électroniques, qui peuvent susciter avant une séparation un état de repos sécurisé du dispositif de perfusion, de préférence déclenché automatiquement par la séparation, et permettre après la jonction une reprise d'une administration programmée, de préférence déclenchée automatiquement par la jonction.

10. Appareil médical portatif selon l'une quelconque des revendications précédentes, **caractérisé en ce que** l'unité d'acheminement (2) est constituée au choix d'une unité d'acheminement pour usage unique ou pour usages multiples.

11. Appareil médical portatif selon l'une quelconque des revendications précédentes, **caractérisé en ce que** l'unité de réservoir (1) est prévue pour un usage unique.

12. Appareil médical portatif selon l'une quelconque des revendications précédentes, **caractérisé en ce qu'**au moins l'adaptateur (3a, 3c) portable sur la peau est prévu que pour un usage unique.
